# EUROPÄISCHE PATENTSCHRIFT

(11) **EP 2 916 815 B1**
(45) Veröffentlichungstag und Bekanntmachung des Hinweises auf die Patenterteilung: **15.08.2018**
(21) Anmeldenummer: 13762723.8
(22) Anmeldetag: 16.08.2013
(51) Int. Cl.: A61K 9/00, A61K 45/06, A61K 31/045, A61K 31/047, A61K 31/19, A61K 47/26, A61K 31/194

(54) **ANTISEPTISCHE ZUSAMMENSETZUNG**
ANTISEPTIC COMPOSITION
COMPOSITION ANTISEPTIQUE

(30) Priorität: 17.08.2012 AT 503242012
(43) Veröffentlichungstag der Anmeldung: 16.09.2015
(73) Patentinhaber: Gebro Holding GmbH, 6391 Fieberbrunn (AT)
(72) Erfinder: HÄUSLER, Franz, 83435 Bad Reichenhall (DE)
(74) Vertreter: Wildhack & Jellinek
(86) Internationale Anmeldenummer: PCT/AT2013/050162
(87) Internationale Veröffentlichungsnummer: WO 2014/026213

(56) Entgegenhaltungen:
- WO-A1-2012/016690
- DE-A1- 1 467 854
- JP-A- 2004 217 264
- US-A- 6 133 327
- DATABASE WPI Week 200049 Thomson Scientific, London, GB; AN 2000-542996 XP002716778, -& WO 00/33815 A1 (TAISHO PHARM CO LTD) 15. Juni 2000 (2000-06-15)
- DATABASE WPI Week 200930 Thomson Scientific, London, GB; AN 2009-H44024 XP002716779, -& CN 101 401 978 A (LIN Y) 8. April 2009 (2009-04-08)
- D. E. Page ET AL: "Vapocoolant spray vs subcutaneous lidocaine injection for reducing the pain of intravenous cannulation: a randomized, controlled, clinical trial", BRITISH JOURNAL OF ANAESTHESIA., vol. 105, no. 4, 3 August 2010 (2010-08-03), pages 519-525, XP55338252, GB ISSN: 0007-0912, DOI: 10.1093/bja/aeq198

## Beschreibung

Die Erfindung betrifft eine antiseptische Kältespray-Zusammensetzung zum Aufbringen auf die menschliche Haut in Form einer topisch applizierbaren Sprayformulierung, enthaltend einen einzigen antiseptischen Wirkstoff bzw. ein einziges Antiseptikum oder eine antiseptische Mischung aus zumindest zwei antiseptischen Wirkstoffen bzw. zwei Antiseptika, sowie ein einziges Kältemittel oder eine Kältemittel-Mischung aus zumindest zwei Kältemitteln, zur vorübergehenden Reduktion der Schmerzempfindlichkeit der Haut, wobei das Kältemittel oder die Kältemittel-Mischung physikalisch durch Verdampfung kühlt und eine Absenkung der Hautoberflächen-Temperatur bewirkt, wobei die antiseptischen Wirkstoffe und die Kältemittel voneinander verschieden bzw. unterschiedliche Substanzen sind.

Bei minimalchirurgischen Eingriffen, insbesondere bei Punktionen der Haut im Rahmen vom Impfungen, Injektionen und beim Anlegen von Infusionen wird das betreffende Hautareal standardmäßig vor dem Eingriff mit Antiseptika behandelt, um die Keimzahl zu reduzieren und dadurch die Infektionsgefahr zu vermindern. Obwohl solche Eingriffe regelmäßig mit einer Schmerzempfindung verbunden sind und obwohl viele Patienten Angst vor dem Eingriff oder der Punktion haben, wird üblicherweise auf eine Schmerzbehandlung verzichtet.

Geeignet zur Schmerzbehandlung wäre insbesondere eine lokalanästhetische Behandlung, bei der das betreffende Hautareal für eine begrenzte Zeit lokal betäubt wird. Durchgeführt wird eine solche Behandlung jedoch bestenfalls bei Kindern oder auf besonderen Wunsch der Patienten. Da sich bei Angst vor Injektionen die naheliegende Betäubung des Behandlungsareals durch Injektion von Lokalanästhetika verbietet, wird die lokalanästhetische Behandlung üblicherweise mit halbfesten Zubereitungen durchgeführt, z.B. EMLA Creme, enthaltend die Lokalanästhetika Lidocain und Prilocain. Allerdings tritt dabei die Wirkung erst nach 30 bis 60 Minuten ein, was beispielsweise für rasche ambulante Behandlungen, insbesondere für Impfungen, oft zu lange ist. Außerdem ist die topische Applikation dieser Lokalanästhetika insofern aufwändig, als die Zubereitung vor dem Eingriff wieder von der Haut entfernt werden muss.

Wenig gebräuchlich, aber aus dem veröffentlichten Stand der Technik bekannt ist es, Kältesprays bzw. Vereisungssprays, z.B. auf Basis von Chlorethan, Freonen oder kurzkettigen Kohlenwasserstoffen, zur Schmerzreduktion einzusetzen, wobei eine Verletzung des Gewebes durch Erfrierungen zu vermeiden ist. Diese Methode der Schmerzreduktion wird deshalb kaum eingesetzt, weil die Schmerzbehandlung nach der antiseptischen Behandlung einen zusätzlichen Arbeitsschritt darstellen würde, und da die handelsüblichen Kältesprays für diese Art der Verwendung wenig geeignet sind. Handelsübliche Kältesprays sind so konzipiert, dass damit auch größere Körperareale bis in tiefere Gewebeschichten gekühlt werden können. Dadurch werden Vasokonstriktion, eine Verringerung des örtlichen Metabolismus und der enzymatischen Aktivität und damit einhergehend eine Reduktion von Entzündung und Schmerz bewirkt. Kältesprays werden deshalb oft zur raschen Erstbehandlung von stumpfen Sportverletzungen vor Ort verwendet. Für die prophylaktische Schmerzreduktion vor Hautpunktionen wäre dagegen eine lokal begrenzte, kurzfristige Abkühlung des Gewebes auf eine Temperatur nötig, bei der die Sensibilität der schmerzempfindlichen Nervenfasern in der Haut reduziert ist.

Es besteht daher ein großes Interesse an der Verfügbarkeit eines Produktes zur Vorbereitung von Nadelstichen oder anderen minimalen iatrogenen Hautverletzungen, das sowohl die Forderung nach Keimreduktion des betroffenen Hautareals als auch die Forderung nach einer Verringerung des Nadelstichschmerzes erfüllt und das die Nachteile der bisherigen Produkte möglichst beseitigt und die Vorteile vereint.

In der DE 1467854 bzw. der FR 96663 wurde versucht dieses Problem zu lösen. Dort wird eine Zerstäuberbombe, in heutiger Nomenklatur eine Druckgaspackung, beschrieben, in der ein Treibmittel enthalten ist, das die Funktion eines lokalanästhesierenden Kältemittels hat, und die zusätzlich ein oder mehrere Antiseptika, unter anderem auch Alkohol enthält. Die in diesen Dokumenten beschriebenen technischen Ausführungsbeispiele sind allerdings in der Praxis untauglich, um den gewünschten schmerzreduzierenden Effekt tatsächlich in hinreichendem Maß zu erreichen. Gemäß der Fachliteratur, z.B. Mawhorter S. et al., J Travel med. 2004 Sep-Oct. 11(5): 267-272, ist es zur Erreichung eines lokalanästhesierenden Effektes nötig, die Haut auf eine Temperatur unter 10 °C abzukühlen. Diese Genze für den Beginn der lokalanästhetischen Wirkung wurde auch durch eigene Untersuchungen der Anmelderin bestätigt. Wegen ihrer geringen Verdampfungsenthalpie lassen sich Hauttemperaturen von unter 10 °C mit den in DE 1467854 bzw. der FR96663 beschriebenen Kältemitteln, nämlich ausschließlich Freonen, nicht bzw. nur für eine unzureichende, extrem kurze Zeit erreichen. Praktische Untersuchungen (siehe später im Detail) haben gezeigt, dass die Zusammensetzungen gemäß der DE 1467854 eine minimale Temperatur von ca. 8 °C erreichen und dass die Temperatur bereits nach einer Sekunde wieder auf einen unwirksamen Wert von > 10 °C ansteigt.

Ein weiterer Nachteil von Freonen ist, dass sie in der Druckgaspackung sehr hohe Drücke aufbauen. Dies führt dazu, dass beim Austritt aus der Ventildüse ein sehr feines Aerosol mit einer geringen mittleren Tröpfchengröße entsteht. Ein durch hohen Druck erzeugtes, sehr feines Aerosol hat zwei entscheidende Nachteile:
Erstens können kleine Tröpfchen den Abstand zwischen der Ventildüse und der Hautoberfläche nur schlecht überwinden, da sie durch den Luftwiderstand im Vergleich zu größeren Tröpfchen überproportional stark gebremst werden und da sie bereits auf dem Weg zur Hautoberfläche teilweise verdampfen. Dadurch gelangt weniger flüssiges Kältemittel auf die Hautoberfläche, was die ohnehin geringe Kältewirkung noch weiter verringert.

Zweitens haben kleine Tröpfchen eine verlängerte Verweilzeit in der Luft und bilden dort ein Aerosol, das durch das medizinische Personal eingeatmet wird. Insbesondere die Tröpfchen unter 20 µm stellen dabei bei chronischer Exposition ein toxikologisches Problem dar (siehe Berger-Preiss E. et al., Int J Hyg Environ Health 2005; 208: 357-372 / Kramer A. et al., BMC Infexct Dis 2007; 7; 117 / Below H. et al., Am J Infect Control 2012; 40; 250-257).

Abgesehen davon sind die in den beiden Patenten genannten Freone inzwischen aus Umweltschutzgründen verboten und Ersatzprodukte aus der gleichen Stoffklasse sind in ihrer Verwendung eingeschränkt.

Die in der DE 1467854 bzw. der FR 96663 beschriebenen Sprays können damit den gewünschten Anforderungen nicht gerecht werden.

Aufgabe der Erfindung ist es daher eine antiseptische Zusammensetzung zu schaffen, die diese Nachteile überwindet, und die sowohl die nötige antiseptische Behandlung eines Hautareals als auch gleichzeitig die erwünschte Schmerzreduktion bei der Punktion leistet, und dies in sehr kurzer Zeit im Sekundenbereich bewerkstelligt, und die zudem einfach und rasch zu applizieren und kostengünstig ist.

Diese Aufgabe wird durch die Merkmale des Anspruchs 1 gelöst. Dabei wird eine antiseptische Zusammensetzung zum Aufbringen auf die menschliche Haut in Form einer topisch applizierbaren Sprayformulierung vorgesehen, enthaltend einen einzigen antiseptischen Wirkstoff oder eine antiseptische Mischung aus zumindest zwei antiseptischen Wirkstoffen, sowie ein einziges Kältemittel oder eine Kältemittel-Mischung aus zumindest zwei Kältemitteln, zur vorübergehenden Reduktion der Schmerzempfindlichkeit der Haut, wobei das Kältemittel oder die Kältemittel-Mischung physikalisch durch Verdampfung kühlt und eine Absenkung der Hautoberflächen-Temperatur bewirkt, wobei die antiseptischen Wirkstoffe und die Kältemittel voneinander verschieden bzw. unterschiedliche Substanzen sind.

Die Zusammensetzung ist geeignet, in unmittelbarer Vorbereitung einer Punktion der Haut, also einer Durchtrennung der Haut, appliziert zu werden.

Überraschenderweise wurde dabei gefunden, dass mit der erfindungsgemäßen Zusammensetzung alle Ziele in einem einzigen Arbeitsgang, auf einfache Weise und zu geringen Kosten erreicht werden können.

Obwohl aus der DE 1467854 bzw. FR 96663 grundsätzlich bekannt, hat sich die Kombination der zwei bekannten Prinzipien der lokalanästhetischen Behandlung mit einem Kältespray und der antiseptischen Behandlung mit einem antiseptischen Spray in der Praxis nie durchgesetzt. Vielmehr standen dieser Kombination mehrere wesentliche Vorurteile entgegen, die durch die Erfindung überwunden werden konnten:
Zur Hautantiseptik vor Punktionen eignen sich insbesondere Alkohole oder Alkohol/Wasser Gemische, da sie bereits nach sehr kurzen Einwirkzeiten eine starke antiseptische Wirkung zeigen. Vorwiegend verwendet werden dafür Isopropanol und Ethanol in konzentrierter Form oder mit einem Zusatz von bis zu 30 % Wasser. Diese Alkohole werden üblicherweise mit einem Tupfer aufgetragen und es war eine weit verbreitete Ansicht, dass dabei das mechanische Abreiben der Haut einen Reinigungseffekt hat und dieser für eine ausreichende antiseptische Wirkung ebenfalls notwendig ist. Weiters herrschte bislang die Ansicht, dass die durch eine kurze Sprayapplikation erreichbare Kontaktzeit von nur wenigen Sekunden zu kurz wäre, um eine ausreichende antiseptische Wirkung zu erzielen. Für den erfindungsgemäßen Spray konnte aber gezeigt werden, dass auch ohne mechanische Reinigung mittels Tupfer und nur durch bloßes Aufsprühen des Antiseptikums auf die Haut eine ausreichende Keimabtötung bewirkt wird.

Betreffend die Kühlwirkung ist bekannt, dass auch handelsübliche Antiseptika in Sprayform beim Auftrag auf die Haut eine gewisse Kühlwirkung erzeugen. Dieses Empfinden einer gewissen kühlenden Wirkung ist aber nicht ausreichend, um zusätzlich auch den gewünschten schmerzreduzierenden Effekt zu erzielen, auf den es jedoch entscheidend ankommt.

Außerdem führen eine Kältebehandlung und die damit einhergehende geringere Gewebetemperatur zur Vasokonstriktion und zu einer Zurückziehung der Gefäße an der behandelten Stelle. Gerade dies ist allerdings für die Durchführung von Venenpunktionen kontraproduktiv und würde das Auffinden einer Vene sowie das Einstechen erschweren. Eine Kältebehandlung vor einer Venenpunktion wurde daher bislang möglichst vermieden. Es hat sich allerdings überraschend gezeigt, dass die erfindungsgemäße Applikation als Spray bedingt durch die Kurzfristigkeit der Behandlung diesen Nachteil gar nicht oder zumindest in sehr viel geringerem Ausmaß als erwartet mit sich bringt.

Die zum Nachweis der Wirksamkeit durchgeführten mikrobiologischen Versuche haben darüber hinaus überraschenderweise ergeben, dass die Kältewirkung der erfindungsgemäßen Zubereitung in mehrfacher Hinsicht die Wirkung des Antiseptikums verstärkt. In jedem Fall zeigte das bloße Aufsprühen des erfindungsgemäßen Sprays die gleiche oder eine bessere antimikrobielle Wirkung als das Auftragen des gleichen Antiseptikums entsprechend dem Stand der Technik mit einem Tupfer.

Weitere vorteilhafte Weiterentwicklungen der Erfindung werden durch die Merkmale der abhängigen Ansprüche beschrieben:
Unter dem Begriff Antiseptik werden antimikrobielle Maßnahmen am oder im lebenden Gewebe verstanden (Wallhäussers: 6. Auflage, Seite 208). Im Unterschied dazu versteht man unter Desinfektion antimikrobielle Maßnahmen an unbelebten Gegenständen, mit Ausnahme der insbesondere chirurgischen Händedesinfektion, da in diesem Fall die Hände als "Werkzeuge" verstanden werden.

Vorliegend werden unter dem Begriff des antiseptischen Wirkstoffs bzw. des Antiseptikums daher antimikrobiell bzw. mikrobizid wirksame Substanzen verstanden, die üblicherweise am oder im lebenden Gewebe, insbesondere auf der menschlichen Haut, verwendet werden.

Um ausreichend schnell und ausreichend gut wirksam zu sein, werden an die antiseptische Wirksamkeit der antiseptischen Wirkstoffe sowie der gesamten erfindungsgemäßen Zusammensetzung gewisse Mindestanforderungen gestellt. Eine sichere Hautantiseptik ist grundsätzlich bei allen durchtrennenden Eingriffen der Haut notwendig (Wallhäussers: 6. Auflage, Seite 212). Entsprechend seinem Verwendungszweck fällt der erfindungsgemäße Spray in die Produktkategorie der "Präoperativen Hautantiseptika". Er muss daher die an ein Hautantiseptikum national und international gestellten Anforderungen hinsichtlich seiner mikrobiziden Wirkung erfüllen und diese Wirkung soll bereits nach einer Einwirkzeit von einer Minute eintreten (Wallhäussers: 6. Auflage, Seite 217).

Die genannten Anforderungen an die antiseptische Wirksamkeit sind unter anderem in den Europäischen Prüfnormen für Desinfektionsmittel und Antiseptika (EN 1040 für bakterizide Wirkung und EN 1275 für fungizide Wirkung) festgelegt. Grundsätzlich wird die Wirksamkeit in einem in-vitro Test (quantitativer Suspensionstest ohne Belastung und mit biotopangepasster Belastung) und in einem in-vivo Test (praxisnahe Prüfung an Probanden) belegt. Dabei ist der in-vivo Test die höherwertige Prüfung; werden die Prüfanforderungen für ein Produkt in-vivo erreicht, ist das Bestehen des in-vitro Tests nicht unbedingt erforderlich. Im Fall der erfindungsgemäßen Spray-Zusammensetzung ist deren Prüfung im in-vitro Test (quantitativer Suspensionstest) aus technischen Gründen nicht möglich, da die Kältemittelkomponente unter den vorgeschriebene Testbedingungen flüchtig ist. Zum Nachweis einer ausreichenden antiseptischen Wirkung der erfindungsgemäßen Zubereitung wird deshalb für die komplette erfindungsgemäße Sprayformulierung in Konformität mit den Prüfnormen nur der in-vivo Test, die praxisnahe Prüfung an Probanden, herangezogen. Zum Nachweis einer ausreichenden antiseptischen Wirkung der erfindungsgemäßen antiseptischen Wirkstoffe bzw. Antiseptika wird dagegen auch der stärker diskriminierende, quantitative Suspensionstest als in-vitro Test herangezogen.

Konkret erfolgt die Überprüfung einer ausreichenden antiseptischen Wirksamkeit gemäß den folgenden Richtlinien: "Standardmethoden der deutschen Gesellschaft für Hygiene und Mikrobiologie (DGHM) zur Prüfung chemischer Desinfektionsverfahren", sowie "Anforderungskatalog für die Aufnahme von chemischen Desinfektionsverfahren in die Desinfektionsmittel-Liste der DGHM".

Eine ausreichende antiseptische Wirksamkeit liegt danach vor, wenn eine Substanz oder eine Formulierung den Test "DGHM Standardmethode 13, Hautdesinfektion - praxisnaher Versuch mit Probanden" besteht, was bedeutet, dass die für die Substanz oder Formulierung gemessenen logarithmischen Reduktionsfaktoren nicht statistisch signifikant kleiner sind als die für ein Referenzprodukt gemessenen. Als Referenzprodukt dient Isopropanol 70 % V/V. Die Substanz oder Formulierung darf also im mikrobiologischen Test auf der Haut beim praxisnahen Versuch mit Probanden gemäß DGHM Standardmethode 13 nicht schlechter abschneiden als 70 %iger Isopropanol.

Gemäß einer für eine ausreichend rasche und ausreichend gute mikrobielle Wirksamkeit besonders vorteilhaften Ausführungsform weisen der antiseptische Wirkstoff bzw. die antiseptische Mischung eine rasche antiseptische Wirksamkeit auf, bei der die Keimreduktion auf der Haut nach 60 Sekunden Einwirkzeit nicht signifikant schlechter ist als eine in einem mit ansonsten gleichen Parametern durchgeführten Referenzverfahren mit 70 Vol.-% Isopropanol nach 60 Sekunden erreichte Keimreduktion bzw. bei der die in einem mikrobiologischen Test auf der Haut, gemäß DGHM Standardmethode 13, Hautdesinfektion - praxisnaher Versuch mit Probanden, gemessenen logarithmischen Reduktionsfaktoren bereits nach 60 Sekunden Einwirkzeit nicht statistisch signifikant kleiner sind als die in einem mit ansonsten gleichen Parametern durchgeführten Referenzverfahren für das Referenzprodukt Isopropanol 70 % V/V nach 60 Sekunden gemessenen Reduktionsfaktoren. Besonders vorteilhaft sind antiseptische Wirkstoffe, bei denen diese Kriterien bereits nach 30 Sekunden Einwirkzeit erfüllt sind.

Dies gilt vorteilhafterweise auch für die erfindungsgemäße Zusammensetzung in ihrer Gesamtheit. Sind diese Kriterien bei der Zusammensetzung erfüllt, ergibt sich eine besonders gute und schnelle mikrobizide Wirksamkeit. Besonders vorteilhaft ist es, wenn diese Kriterien bereits nach einer Einwirkzeit von 30 Sekunden erfüllt sind.

Der bzw. die antiseptische(n) Wirkstoff(e) ist/sind vorteilhafterweise ausgewählt aus kurzkettigen, weniger als 7 C-Atomen enthaltenden, einwertigen Alkoholen, insbesondere aus den 2 oder 3 C-Atomen enthaltenden einwertigen Alkoholen. Vor allem sind Ethanol, Propan-1-ol und/oder Propan-2-ol (Isopropanol) vorteilhaft einsetzbar. Anspruch 1 offenbart Propan-1-ol und Propan-2-ol (Isopropanol) als antiseptische Wirkstoffe. Im Sinne der Patentanmeldung werden als Kältemittel Stoffe bezeichnet, die einerseits physiologisch verträglich sind und weiters nach Aufbringung auf die menschliche Haut rasch verdampfen und dabei die Hautoberfläche durch die entstehende Verdunstungskälte abkühlen. Die erfindungsgemäßen Kältemittel bewirken also eine tatsächliche, physikalische Abkühlung der Haut. Stoffe vom Wirkungstyp des Menthols oder Camphers, die auf chemischem Weg die Kälterezeptoren der Haut reizen und auf diese Weise ein subjektives Kältegefühl erzeugen, das nicht mit einer Absenkung der Hauttemperatur verbunden ist, sind keine Kältemittel im Sinne der Erfindung.

Für den angestrebten lokalanästhesierenden Effekt ist es nötig, die Haut auf eine Temperatur von wenigstens 10 °C abzukühlen. Bei eirer zu starken Abkühlung, insbesondere auf Temperaturen unterhalb von 0 °C besteht die Gefahr von Kälteschäden der Haut. Der erfindungsgemäßen Spray ist demnach besonders gut einsetzbar und besitzt eine ausreichend schmerzreduzierend-kühlende Wirkung bei minimaler Gefahr einer Gewebebeschädigung durch Vereisung, wenn nach einer 2 Sekunden andauernden Sprayapplikation der Zusammensetzung, bei der auf einen Hautbereich am Unterarm mit einer Größe von 10 cm² eine Menge von 200 mg der Zusammensetzung pro cm² aufgetragen wird, eine mit einem Infrarot Temperatursensor gemessene Abkühlung der Hautoberfläche auf eine Temperatur zwischen 10° und -5°C, insbesondere zwischen 5° und 0° C, bewirkbar ist. Diese Abkühlung tritt bereits während der Applikation auf bzw. die Hauttemperatur wird unmittelbar nach Ende des Sprühvorgangs, maximal bis zu 1 sek bzw. innerhalb von +/-1 sek nach Ende des Sprühvorgangs gemessen.

Die tiefe Temperatur von unter 10 °C wird auch mit einem Kältespray nur für eine sehr kurze Zeit während und unmittelbar nach dem Sprühen erreicht. Bei einer Abkühlung der Haut auf einen Wert von + 5 °C bis 0 °C beobachtet man innerhalb von 10 Sekunden einen Wiederanstieg der Hautoberflächentemperatur auf etwa 20 °C. Es wurde jedoch überraschend gefunden, dass ein subjektives Kältegefühl und der lokal anästhesierende, schmerzreduzierende Effekt nach einer Abkühlung der Haut auf unter 10 °C noch für bis zu 30 - 60 Sekunden anhält, auch wenn sich die Haut während dieser Zeit bereits wieder erwärmt hat. Diese Zeitspanne reicht aus, um die Punktionen der Haut in Zusammenhang mit Injektionen, Infusionen und Impfungen bei ausreichender Reduktion der Schmerzempfindung durchzuführen.

Die in den erfindungsgemäßen Formulierungen als Antiseptika besonders bevorzugt verwendeten aliphatischen Alkohole besitzen eine Siedetemperatur von zwischen + 50 °C und + 100 °C. Sie bewirken daher auch gewisse kühlende Empfindungen. Da sie eine relativ hohe Siedetemperatur, aber auch eine hohe Verdampfungsenthalpie haben, haben sie zwar nur eine geringe Kühlwirkung, die regelmäßig nicht ausreicht, um die angestrebte Oberflächentemperatur der Haut von < 10 ° C zu erreichen, sie tragen jedoch zu einer Verlängerung der Kühlwirkung bei.

Umgekehrt haben auch viele Kältemittel gewisse antiseptische Eigenschaften, die wiederum nicht ausreichen, die geforderte mikrobizide Wirkung zu erzielen.

In den erfindungsgemäßen Formulierungen werden die aliphatischen Alkohole deshalb zwingend mit stärker wirkenden Kältemitteln kombiniert, die einen Siedepunkt unterhalb der physiologischen Körpertemperatur von + 37 °C und meist auch eine geringere Verdampfungsenthalpie haben. Diese führen zu einer kurzfristigen, aber starken Abkühlung der Haut.

Die Kühlwirkung und die physiologische Verträglichkeit der erfindungsgemäßen Zusammensetzung hängen zum einen vom Siedepunkt bzw. Dampfdruck zum anderen von der Verdampfungsenthalpie der Kältemittel oder der Kältemittel-Mischung ab. Die Kühlwirkung beruht dabei im Wesentlichen darauf, dass der Haut Energie entzogen wird, die bei der Verdampfung des Kältemittels verbraucht wird. Es gibt einen groben, aber nicht linearen Zusammenhang zwischen Verdampfungsenthalpie und Siedepunkt dergestalt, dass Kältemittel mit hohem Siedepunkt meist auch eine hohe Verdampfungsenthalpie haben und umgekehrt.

In diesem Zusammenhang ist es vorteilhaft für die schmerzreduzierende Wirkung, wenn das einzige Kältemittel oder die Kältemittel-Mischung eine Siedetemperatur zwischen +20 °C und -20 °C aufweist. Dabei ist vorteilhafterweise vorgesehen, dass die einzelnen Komponenten einer Kältemittel-Mischung jeweils eine Siedetemperatur zwischen +37 °C und -50 °C aufweisen.

Eine gute kühlende Wirkung lässt sich erzielen, wenn das einzige Kältemittel oder die Kältemittel-Mischung eine molare Verdampfungsenthalpie Δ_{V}H⁰ zwischen 10 und 35 kJ/mol, vorzugsweise zwischen 15 und 30 kJ/mol, besitzt.

Eine besonders gute kühlende Wirkung lässt sich erzielen, wenn das einzige Kältemittel oder die Kältemittel-Mischung eine bei 295 K gemessene massenbezogene Verdampfungsenthalpie von mindestens 250 kJ/kg besitzt. Wie nachfolgende Tabelle zeigt, lässt sich die geforderte Mindestverdampfungsenthalpie von 250 kJ/kg (bei 295 K) durch Mischungen von Alkanen sicher erreichen, nicht aber durch die Mischung von Freonen:

**Tabelle Verdampfungsenthalpien (Hv):**

| | Hv kJ/kg |
|---|---|
| Butan | 406 (295 K) |
| Pentan | 365 (295 K) |
| Freon 11 | 183 (295 K) |
| Freon 12 | 150 (295 K) |
| Freon 134 | 217 (295 K) |

Eine vorteilhaft einsetzbare antiseptische Zusammensetzung liegt vor, wenn das/die Kältemittel ein verflüssigtes Treibgas, insbesondere ein Flüssiggas aus leicht verflüssigbaren Kohlenwasserstoff-Verbindungen, ist und/oder das/die Kältemittel ausgewählt ist/sind aus der Gruppe von n-Propan, n-Butan, iso-Butan, n-Pentan, n-Hexan, Chlorethan oder Distickstoffmonoxid. Besonders einfach ist es, wenn das einzige Kältemittel oder die Kältemittel-Mischung gleichzeitig auch als Treibmittel fungiert.

Besonders bevorzugt ist es, wenn das/die Kältemittel nur aus Alkanen bestehen.

Besonders bevorzugt ist es, wenn die Kältemittel bzw. die Zusammensetzung völlig frei von Freonen bzw. halogenierten, insbesondere fluorierten, Kohlenwasserstoffen ist. FKWs haben eine für die erfindungsgemäße Anwendung zu geringe Verdampfungsenthalpie und darüber hinaus besitzen alle FKWs ein erhebliches Treibhauspotential und ihre Verwendung ist gesetzlich eingeschränkt.

Besonders bevorzugt ist es, wenn das Kältemittel eine Mischung von n-Pentan und n-Butan ist, insbesondere in einem Gew.-%-Verhältnis von n-Pentan : n-Butan von 15-25 : 45-55, vorzugsweise 2 : 5.

In diesem Zusammenhang ist es vorteilhaft, wenn die Zusammensetzung ausschließlich aus Isopropanol, n-Pentan und n-Butan besteht, wobei Isopropanol vorzugsweise mit ca. 25 - 35 Gew %, n-Pentan mit ca. 15 - 25 Gew % und n-Butan mit ca. 45 - 55 Gew %, enthalten ist. Besonders vorteilhaft ist eine Zusammensetzung von Isopropanol 30 Gew %, n - Pentan 20 Gew % und n - Butan 50 Gew %.

Gegebenenfalls kann zusätzlich auch noch n-Propan in einer Menge von ca. 5 bis 10 Gew.% enthalten sein, wobei sich dann die Menge von n - Butan entsprechend reduziert.

Die folgende Tabelle führt exemplarisch die Siedetemperaturen und die Verdampfungsenthalpien einiger typischer, geeigneter Kältemittel auf:

**Tabelle 1 - Siedetemperaturen und Verdampfungsenthalpien einiger Kältemittel**

| Kältemittel | Siedepunkt | Verdampfungsenthalpie | |
|---|---|---|---|
| | (°C) | (kJ/mol) | (kJ/kg) |
| Propan | - 42 | 19 | 431 |
| n-Butan | - 0,5 | 23,6 | 406 |
| n-Pentan | + 36 | | 365 |
| Chlorethan | + 12,3 | 25,5 | 395 |
| 2-Propanol (zum Vergleich) | + 82 | 40 | 666 |
| Wasser (zum Vergleich) | + 100 °C | 40,8 | 2257 |

Die Kältemittel können ausgewählt werden aus der Gruppe der homologen, isomeren und cyclischen Alkane, insbesondere alle Isomere von Propan, Butan, Pentan, Hexan, 2,2-Dimethylbutan. Ethan hat mit - 89 °C einen so tiefen Siedepunkt, dass das Risiko von Kälteschäden auf der Haut besteht. Propan mit einem Siedepunkt von - 42 °C kann aus gleichem Grund nur in relativ geringer Konzentration verwendet werden. n-Heptan hat einen Siedepunkt von + 98 °C und ist als Kältemittel bereits nicht mehr geeignet. Das gleiche gilt auch für 2-Propanol oder Wasser.

Die Kältemittel können weiters ausgewählt werden aus der Gruppe der druckverflüssigten Gase, insbesondere Kohlendioxid, Distickstoffmonoxid;
oder weiters aus der Gruppe der Ether, insbesondere Methylether, Ethylether, Tetrahydrofuran, 1,4-Dioxan. Ether haben zudem einen die aseptische Wirkung verstärkenden Effekt, ohne selbst antimikrobiell wirksam zu sein. Sie erhöhen die Membrandurchlässigkeit der Zellwände von Keimen.

Gemäß einer besonders vorteilhaften Ausgestaltung der Erfindung, ist vorgesehen, dass ein oder mehrere, die antiseptische Wirkung verstärkende, Wirkverstärker enthalten sein können, wobei der/die Wirkverstärker von den antiseptischen Wirkstoffen und den Kältemitteln unterschiedlich ist/sind. Die Ansprüche offenbaren Wasser als Wirkverstärker. Als Wirkverstärker werden vorliegend Substanzen verstanden, die die mikrobizide Wirkung verstärken, beispielsweise indem sie die das Eindringen der antimikrobiellen Alkohole in die Zellwand der Mikroorganismen erleichtern, selbst aber entweder gar keine oder keine ausreichende mikrobizide Wirkung haben, also selbst keine antiseptischen Wirkstoffe im Sinne der Erfindung sind.

Hierbei ist vorteilhafterweise vorgesehen, dass der/die Wirkverstärker als solche eine antiseptische Wirksamkeit aufweist(en), bei der die Keimreduktion auf der Haut nach 60 Sekunden Einwirkzeit signifikant schlechter ist als eine in einem mit ansonsten gleichen Parametern durchgeführten Referenzverfahren mit 70 Vol.-% Isopropanol nach 60 Sekunden erreichte Keimreduktion bzw. bei der die in einem mikrobiologischen Test auf der Haut, gemäß DGHM Standardmethode 13, Hautdesinfektion - praxisnaher Versuch mit Probanden, gemessenen logarithmischen Reduktionsfaktoren nach 60 Sekunden Einwirkzeit statistisch signifikant kleiner sind als die in einem mit ansonsten gleichen Parametern durchgeführten Referenzverfahren für das Referenzprodukt Isopropanol 70 % V/V nach 60 Sekunden gemessenen Reduktionsfaktoren. Der/die Wirkverstärker erfüllen also nicht die Anforderungen für die rasche und qualitative mikrobielle Wirksamkeit des antiseptischen Wirkstoffs der Zusammensetzung.

Gemäß einer weiteren vorteilhaften Ausführungsform ist vorgesehen, dass der/die Wirkverstärker selbst überhaupt keine bzw. keinen nennenswerte antiseptische Wirksamkeit besitzt(en), insbesondere nicht in den Konzentrationen, in denen er in der Zusammensetzung enthalten ist bzw. die auf der Haut aufgetragen werden.

Die Wirkverstärker können vorteilhaft ausgewählt werden aus der Gruppe der aliphatischen Alkohole, insbesondere alle Isomere von Butanol, Pentanol, Hexanol; der mehrwertigen Alkohole, insbesondere Propan-1,2-diol, Glycerol, Butan-1,3-diol, Octoxyglycerin; oder der aromatischen Alkohole, insbesondere Benzylalkohol, Dichlorbenzylakohol, Phenylethanol, 2-Phenoxyethanol, 1-Phenoxy-2-Propanol;
weiters aus der Gruppe der Aldhyde und Ketone, insbesondere Formaldyd, Glyoxal, Zimtaldehyd, Aceton;
weiters aus der Gruppe der anorganischen Säuren, insbesondere Borsäure, Borax, Phosphorsäure, saure Phosphate, Phosphonsäure, Salzsäure, Salpertersäure, Schwefelsäure, Dihydrogensulfit;
weiters und besonders vorteilhaft aus der Gruppe der organischen Säuren und ihre sauren Salze, insbesondere Essigsäure, Propionsäure, Milchsäure, Äpfelsäure, Glycolsäure, Sorbinsäure, Benzoesäure, Salicylsäure, 4-Hydroxybenzoesäure-ester, 4-Hydroxybenzoesäure-benzylester, Ascorbinsäure, Fumarsäure, Adipinsäure, Zitronensäure, Bernsteinsäure, Weinsäure, Mandelsäure, Glutarsäure;
weiters aus der Gruppe der Oxidantien, insbesondere Wasserstoffperoxid, Peressigsäure, Hypochlorite, Jod / Povidon-Jod, Kaliumpermanganat;
weiters aus der Gruppe der Phenole, insbesondere Phenol, Chlorocresol, 4-Chlor-3,5-dimethylphenol, Thymol, Eugenol, 2-Phenylphenol, Butylhydroxytoluol, Triclosan;
weiters aus der Gruppe der oberflächenaktiven Substanzen, insbesondere Benzalkoniumchlorid, Benzethoniumchlorid, Mecetroniumetilsulfat, Cetrimid, Cetylpyridiniumchlorid, Nichtionogene Tenside (z.B. Tween 80);
weiters aus der Gruppe der Guanidine/Biguadinidine, insbesondere Polihexanid, Chlorhexidin und -salze;
weiters aus der Gruppe der N-Heterocyclen, insbesondere Octenidindihydrochlorid, Hexetidin, Decapinol;
weiters aus der Gruppe von Dimethylsulfoxid, Chlorbutanol, Triclocarban, 8-Quinolinol, Clioquinol, Parachlormetaxylenol, Nitrofurazon, Thiocyanate, Metallverbindungen enthaltend Silber, Quecksilber oder Zink, Sulfonamide, Antibiotika, Metronidazol, Topische Virustatika, Topische Antimykotikader;
weiters aus der Gruppe der Naturstoffe und Naturstoffgemische, insbesondere etherische Öle, z.B. Zimt, Nelke, Rosmarin;
weiters kann Wasser als Wirkverstärker verwendet werden.

Besonders bevorzugt sind die Wirkverstärker ausgewählt aus der Gruppe von Äpfelsäure, Milchsäure, Essigsäure, Weinsäure, Gyloxal, 1,2-Propandiol, 1,3-Butandiol, Dimethylether, etherischen Ölen oder Die Ansprüche offenbaren Wasser als Wirkverstärker. Das Mengenverhältnis zwischen Antiseptikum und Kältemittel ist für die Funktionalität des Produktes insofern von Bedeutung, da in einer bestimmten Zeiteinheit gleichzeitig geeignete Mengen an Antiseptikum und an Kältemittel freigesetzt werden müssen.

Eine typische Sprühsituation ist dadurch gekennzeichnet, dass aus einer Entfernung von ca. 10 cm ein Hautareal von ca. 10 cm² besprüht wird. Um auf einem Hautareal von 10 cm² eine ausreichende keimtötende Wirkung zu erzielen, ist zu empfehlen, dass etwa 0,1 - 0,3 g eines Antiseptikums aufgetragen werden. Auch bei einem sehr wirksamen Antiseptikum lässt sich die Auftragsmenge nicht wesentlich unter 0,1 g senken, da andernfalls keine vollständige Benetzung der Fläche von 10 cm² stattfindet und die Mittel nur bei direktem Kontakt wirken.

Die benötigte Menge an Kältemittel hängt von der spezifischen Kühlwirkung des Kältemittels ab. Bei zu geringer Kühlwirkung kommt es nicht zur gewünschten Reduktion der Schmerzempfindlichkeit im besprühten Hautareal. Die Haut muss hierfür oberflächlich zumindest auf eine Temperatur von 10 °C abgekühlt werden. Bei zu großer Kältewirkung kann es allerdings zu Kälteschäden der Haut kommen. Diese können von einer Rötung, über eine Quaddelbildung bis zu Erfrierungen reichen.

Auf Grund dieser Randbedingungen ergeben sich die für die Praxis vorteilhaften quantitativen Zusammensetzungen der erfindungsgemäßen Formulierung.

Der/die antiseptischen Wirkstoff(e) sind vorteilhafterweise in einer Menge von insgesamt 10 bis 30 Gew.-%, bezogen auf die gesamte Zusammensetzung, enthalten.

Das/die Kältemittel sind vorteilhafterweise in einer Menge von insgesamt 70 bis 90 Gew.-%, bezogen auf die gesamte Zusammensetzung, enthalten.

Der/die Wirkverstärker sind vorteilhafterweise in einer Menge von insgesamt 0,1 - 10 Gew.-%, bezogen auf die Menge der antiseptischen Wirkstoffe, enthalten.

Bekannt ist, dass der Zusatz von Wasser die antimikrobielle Wirkung von Isopropanol verstärkt. Eine sehr gute antimikrobielle Wirkung zeigt eine Mischung aus 70 % Isopropanol und 30 % Wasser und diese Mischung ist deshalb als Antiseptikum weit verbreitet. Überraschenderweise konnte gezeigt werden, dass die Kombination von Isopropanol mit dem Kältemittel eine bessere antimikrobielle Wirkung hat als die bekannte und oben genannte Kombination von Isopropanol mit Wasser im Verhältnis 7:3.

Besonders vorteilhaft ist es, wenn der/die antiseptischen Wirkstoff(e), das/die Kältemittel und/oder der/die Wirkverstärker, insbesondere alle Komponenten, untereinander vollständig oder nahezu vollständig mischbar sind. Alternativ sind aber auch zweiphasige Systeme bzw. Aerosol-Emulsionen möglich.

Vorteilhaft ist es auch, wenn die Zusammensetzung während und nach der Applikation flüssig und nicht schäumend ist.

Gemäß einer weiteren vorteilhaften Ausgestaltung der Erfindung ist vorgesehen, dass die Zusammensetzung frei ist von weiteren Exzipienten, Hilfsstoffen und/oder Zusatzstoffen, insbesondere frei von viskositätserhöhenden und/oder filmbildenden Polymeren, Stärken, Zuckern, Zuckeralkoholen, Fetten, Ölen, Wachsen, Aromastoffen und/oder längerkettigen, insbesondere geradkettigen, Fettsäuren mit mehr als 10 C-Atomen. Auf diese Weise wird eine einfache und kostengünstige Zusammensetzung erhalten und bei der Durchtrennung der Haut hinderliche und kontraindizierte Rückstände, die nach der Applikation auf der Haut verbleiben, werden verringert bzw. vermieden.

Insbesondere vorteilhaft ist es, wenn die Zusammensetzung frei von weiteren therapeutisch wirksamen Bestandteilen, insbesondere frei von Antibiotika, Antimykotika und/oder Virostatika oder Viruziden, ist. Auf diese oft teuren Substanzen kann verzichtet werden, da die Wirkungen des Sprays bereits ausreichend sind. Außerdem würde die Verwendung dieser Substanzen das Risiko von unerwünschten Nebenwirkungen und die Gefahr der Resistenzbildung vergrößern.

Eine vorteilhafte Variante sieht vor, dass der Gehalt an Rückständen nach der Verdampfung nach der Sprayapplikation maximal 4 Gew.-%, vorzugsweise maximal 2 Gew.-%, bezogen auf die gesamte Zusammensetzung, beträgt.

In diesem Zusammenhang ist es besonders vorteilhaft, wenn die Zusammensetzung nach der Sprayapplikation auf der menschlichen Haut völlig rückstandsfrei verdampft bzw. dass in der Zusammensetzung ausschließlich rückstandsfrei verdampfende Stoffe enthalten sind.

Dadurch wird gewährleistet, dass möglichst wenige, vorzugsweise gar keine unerwünschten Substanzen in die durch die Punktion verursachte Wunde gelangen können und die Gefahr von Irritationen wird verringert.

Bei einer besonders vorteilhaften Ausführungsform ist vorgesehen, dass die Zusammensetzung bei Raumtemperatur (25 °C) in zumindest teilweise flüssiger Form, in einem Behältnis bei einem gegenüber dem Umgebungsdruck (Normaldruck) nur mäßig erhöhten Druck vorliegt. Insbesondere vorteilhaft liegt die Zusammensetzung in druckverflüssigter Form in einer Druckgaspackung bzw. als Druckgassystem, beispielsweise in einer form- und druckstabilen Sprühdose, oder in einem BOV (Bag-on-Valve)-System vor, wobei der Innendruck des Behältnisses im Bereich zwischen 1 und 2 bar liegt.

Die im Spray verwendeten Kältemittel stehen in der Sprühdose unter Druck und liegen ganz oder teilweise in flüssiger Form vor. Nach dem Austritt des Kältemittels durch das Dosenventil und insbesondere beim Auftreffen auf die körperwarme Haut kommt es zu einer Expansion und/oder zu einem Verdampfen des Kältemittels. Dabei wird der Umgebung Energie entzogen, was zu der gewünschten Temperaturabsenkung und zur schmerzstillenden kühlenden Wirkung führt.

Die Dose wird durch ein Ventil gasdicht verschlossen, das erst bei Betätigung den Inhalt der Dose frei gibt. Dabei kommt es zu einer Zerstäubung des flüssigen Inhalts. Dieser Effekt ist für die notwendige feine und gleichmäßige Verteilung des Antiseptikums wichtig und im Falle des erfindungsgemäßen Sprays ermöglicht die Zerteilung in kleine Tropfen mit einer großen spezifischen Oberfläche darüber hinaus die schnelle Verdampfung des Inhalts und damit einen starken Abkühleffekt. Dabei soll jedoch die Bildung eines zu feinen Aerosols, insbesondere eines Aerosols mit einer Tröpfchengröße von < 20 µm vermieden werden, da der Kältemittelanteil so kleiner Tröpfchen dazu neigt, schon vor dem Auftreffen auf der Haut zu verdampfen, was die Kühlwirkung verschlechtert, und da der Alkoholanteil der Tröpfchen eine verlängerte Verweilzeit in der Umgebungsluft hat und deshalb leicht eingeatmet werden kann, was zu toxikologischen Problemen insbesondere für das medizinische Personal, das den Spray häufig anwendet, führen kann. Die sich bildende Tröpfchengröße ist damit vorteilhafterweise größer als 20 µm, vorzugsweise größer als 50 µm.

Es gibt verschiedene Sprühsysteme, die sich im Wesentlichen durch Ihre Ventiltechnik unterscheiden. Insbesondere kommen folgende Sprühsysteme in Frage:
Dose mit Standardventil: Der Doseninhalt erreicht das Ventil über ein Steigrohr. Je nachdem ob ein kurzes oder ein langes Steigrohr verwendet wird, kann die Dose nur in aufrechter oder in über Kopf Position verwendet werden.

Dose mit Dosierventil: Ein Dosierventil gibt pro Sprühstoß eine definierte Menge an Produkt ab.

Dose mit Kugelventil: Dieses Ventil arbeitet sowohl in aufrechter als auch in über Kopf Position, ist aber in horizontaler Lage nur begrenzt einsetzbar.

BOV-System: Im BOV System befindet sich das Sprühmedium in einem Beutel, der an das Ventil angeschlossen ist. Der Sprühdruck wird über ein zusätzliches Druckgas aufgebaut, das sich zwischen der Behälterwand und dem Beutel befindet. Als Druckgas können z.B. Stickstoff, Kohlendioxid, Distickstoffoxid, alle Edelgase oder Pressluft verwendet werden. Das BOV System ist in jeder Sprühposition anwendbar.

Die Füllmenge pro Druckgaspackung ist für die Wirksamkeit ohne Bedeutung. Von praktischem Interesse sind Füllmengen zwischen 50 ml und 250 ml. Für eine Einzelanwendung wird etwa eine Menge von 1 ml benötigt, so dass diese Menge als unterste Grenze der Füllmenge anzusehen wäre. Größere Druckgaspackungen wären theoretisch möglich, schon ab 500 ml wären aber die Dosen unpraktisch in der Handhabung. Stationäre Flaschensysteme mit einer Entnahme über einen druckfesten Schlauch wären denkbar.

Die Erfindung betrifft weiters die vorteilhafte Anwendung der erfindungsgemäßen antiseptischen Zusammensetzung zur Kühlung, Desinfektion und lokalen Reduktion der Schmerzempfindlichkeit eines Hautareals, insbesondere maximal 60, insbesondere vorteilhaft 30, Sekunden vor einer Punktion der Haut.

Insbesondere vorteilhaft ist dabei die Verwendung der erfindungsgemäßen antiseptischen Zusammensetzung, gegebenenfalls zur Herstellung eines Arzneimittels, zur Vorbeugung und Vermeidung von Nadelstichschmerzen durch topische Sprayapplikation auf die Haut maximal 60, insbesondere vorteilhaft 30, Sekunden vor einer Punktion. Die Erfindung betrifft weiters die augenfällige Herrichtung der Zusammensetzung, wobei die Zusammensetzung insbesondere zur topischen Verabreichung als Spray bzw. Sprayapplikation auf die Haut maximal 60, insbesondere vorteilhaft 30, Sekunden vor einer Punktion bzw. Sprayapplikation hergerichtet ist. Die medizinische und physikalische Eignung der erfindungsgemäßen Zusammensetzung zur Sprayapplikation in unmittelbarer Vorbereitung einer die Haut durchtrennenden Punktion, ist zu jeder Zeit gegeben.

Vorteilhafterweise wird die Zusammensetzung durch eine 1 - 5 Sekunden, vorzugsweise 2 Sekunden, andauernde Sprayapplikation auf einen lokalen Hautbereich, insbesondere mit einer Größe von 5 - 20 cm², vorzugsweise ca. 10 cm², aufgebracht und dabei insgesamt eine Menge von 150 bis 250 mg der Zusammensetzung / cm² aufgetragen.

### Beispiele:

Im Folgenden wird die Erfindung an Hand von einigen exemplarischen und nicht einschränkend zu verstehenden Ausführungsbeispielen beispielhaft dargestellt: Die Formulierungen 4, 5 und 10 sind als Referenzformulierungen zu verstehen. Formulierungsbeispiele der Zusammensetzung ohne Wirkverstärker:
Formulierung 1:

| | |
|---|---|
| Isopropanol | 30 % |
| | |
| n - Pentan | 20 % |
| n - Butan | 44 % |
| n - Propan | 6 % |

Formulierung 2: mit dieser Formulierung wurden auch die weiteren Tests zur Kältewirkung vorgenommen

| | |
|---|---|
| Isopropanol | 30 % |
| | |
| n - Pentan | 20 % |
| n - Butan | 50 % |

Formulierung 3:

| | |
|---|---|
| Isopropanol | 10 % |
| | |
| n - Pentan | 25 % |
| n - Butan | 45 % |
| iso - Butan | 15 % |
| n - Propan | 5 % |

Formulierung 4:

| | |
|---|---|
| Ethanol | 5 % |
| | |
| n - Pentan | 25 % |
| n - Butan | 50 % |
| iso - Butan | 10 % |
| Chlorethan | 10 % |

Formulierung 5:

| | |
|---|---|
| n-Propanol | 50 % |
| | |
| n - Pentan | 15 % |
| n - Butan | 25 % |
| iso - Butan | 55 % |
| n - Propan | 5 % |

Formulierungsbeispiele der Zusammensetzung mit Wirkverstärker(n):
Formulierung 6:

| | |
|---|---|
| Isopropanol | 20 % |
| Ethanol | 6,3 % |
| Wasser | 2,7 % |
| | |
| n - Pentan | 9,5 % |
| n - Butan | 47,5% |
| iso - Butan | 7 % |
| n - Propan | 7 % |

Formulierung 7: mit dieser Formulierung wurden auch die weiteren Tests vorgenommen

| | |
|---|---|
| Isopropanol | 29 % |
| Äpfelsäure | 1 % |
| | |
| n - Pentan | 20 % |
| n - Butan | 44 % |
| n - Propan | 6 % |

Formulierung 8:

| | |
|---|---|
| Isopropanol | 25 % |
| 1,3-Butandiol | 5 % |
| | |
| n - Pentan | 15 % |
| n - Butan | 45 % |
| iso - Butan | 5 % |
| n - Propan | 5 % |

Formulierung 9:

| | |
|---|---|
| n-Propanol | 22 % |
| Dimethylether | 3 % |
| | |
| Distickstoffmonoxid | 30 % |
| n - Butan | 25 % |
| n - Propan | 20 % |

Formulierung 10:

| | |
|---|---|
| Ethanol | 19,6 % |
| Wasser | 8,4 % |
| Milchsäure | 2 % |
| n - Pentan | 15 % |
| n - Butan | 45 % |
| iso - Butan | 5 % |
| n - Propan | 5 % |

Formulierung 11:

| | |
|---|---|
| Isopropanol | 10 % |
| n - Propanol | 3 % |
| 1,3-Butanol | 1 % |
| Weinsäure | 1 % |
| | |
| n - Hexan | 5 % |
| n - Pentan | 10 % |
| n - Butan | 50 % |
| iso - Butan | 10 % |
| n - Propan | 10 % |

Die angeführten Formulierungsbeispiele umfassen jeweils nur die Zusammensetzung, die beim Betätigen des Sprühdosenventils aus der Dose ausgetrieben wird. Diese Zusammensetzungen können in eine Dose mit Standardventil gefüllt werden. In diesem Fall bilden die in der Dose druckverflüssigten Kältemittel gleichzeitig das Treibgas, das für die Entleerung der Dose durch das Ventil sorgt. Falls die Mischungen in eine Dose mit BOV-System gefüllt werden, wird zusätzlich ein komprimiertes Treibgas zwischen der Dosenwand und dem Innenbeutel benötigt.

### Nachweise für die Wirksamkeit:

### Mikrobielle Wirksamkeit der erfindungsgemäßen Zusammensetzung:

Die relevantesten Ergebnisse der durchgeführten mikrobiologischen Tests zur Beurteilung der mikrobiellen Wirksamkeit der erfindungsgemäßen Zusammensetzung sind nachfolgend beschrieben:
Hierbei wurde die mikrobielle Wirksamkeit einer beispielhaften erfindungsgemäßen Zusammensetzung (Formulierung 7, appliziert mit Druckgas) im Vergleich zu 70 % Isopropanol (= Referenz gemäß DGHM, appliziert mit Tupfer), zu reinem kühlenden Treibmittel (Formulierung 7x, entsprechend Formulierung 7 ohne Isopropanol und Äpfelsäure, appliziert mit Druckgas) und zu 100 % Isopropanol (appliziert als Pumpspray) getestet.

Der Test wurde nach der Methode der Deutschen Gesellschaft für Hygiene und Mikrobiologie (DGHM) an 10 Probanden auf dem Oberarm durchgeführt. Beim Oberarm handelt es sich um ein talgdrüsenarmes Hautareal; dieses entspricht dem für die klinische Anwendung des Sprays vorzugsweise genutzten Hauttyp.

Im Fall des Referenzprodukts wurde der 70 %ige Isopropanol mit einem Tupfer aufgetragen und das Hautareal für die Einwirkzeit mittels des Tupfers feucht gehalten. Im Fall der beiden Druckgaspackungen (Formulierung 7 und Formulierung 7x ohne Isopropanol und Äpfelsäure) wurde die jeweilige Zubereitung aus einer Entfernung von 10 cm für 2 sec aufgesprüht. Die Auftragsmenge betrug insgesamt je ca. 0,18 g/cm². Im Fall des Pumpsprays wurden aus einer Entfernung von 10 cm zwei Pumpstöße aufgebracht. Die Auftragsmenge betrug insgesamt ca. 0,21 g/cm².

Die Ergebnisse sind in Form logarithmischer Reduktionsfaktoren (RF-Werte) angegeben. Sie stellen die Abnahme der Gesamtkeimzahl auf der Haut bezogen auf die natürliche Hautflora dar. Folgende RF-Werte (n=10) wurden gefunden:

**Tabelle 2 - Mikrobielle Wirksamkeit**

| Einwirkzeit | Referenzprodukt | Testprodukte | | |
|---|---|---|---|---|
| | 70% Isopropanol (Tupfer) | Formulierung 7x (Druckgas) | 100% Isopropanol (Pumpspray) | Formulierung 7 (Druckgas) |
| 10 s | | 0,37 | 0,93 | 1,16 |
| 15 s | 1,26 | | | |
| 20 s | | 0,63 | 0,90 | 1,49 |
| 30 s | 1,30 | | | |
| 60 s | 1,27 | | | |

Entsprechend der Forderung der DGHM besteht ein Testprodukt den Test, wenn der erzielte RF-Wert nicht statistisch signifikant kleiner ist als der des Referenzproduktes (70 % Isopropanol). Das Testprodukt muss also im Vergleich zum Referenzprodukt gleich gut oder besser sein.

Die RF-Werte für das Referenzprodukt (70% Isopropanol) zeigen, dass die volle Wirksamkeit des Referenzproduktes bereits nach 15 sec gegeben ist und danach keine weitere Abnahme der Keimzahl eintritt.

Für die erfindungsgemäße Zusammensetzung gemäß Formulierung 7 konnte bereits nach einer Einwirkzeit von 10 sec eine den DGHM Richtlinien konforme mikrobielle Wirksamkeit gezeigt werden. Diese erhöhte sich bei einer Einwirkzeit von 20 sec sogar noch weiter. Der für Formulierung 7 nach 10 Sekunden gemessene numerische Reduktionsfaktor 1,16 war statistisch nicht signifikant kleiner als der für das Referenzprodukt nach 15 Sekunden gemessene Reduktionsfaktor von 1,26. Der Vergleich zwischen der aus einem Druckgassystem lediglich aufgesprühten erfindungsgemäßen Formulierung mit dem mittels Tupfer aufgetragenen Referenzprodukt zeigte auch, dass die vermeintlichen Vorteile des Auftrags mittels Tupfer, nämlich die Verwendung eines Überschusses an Antiseptikum, das Feuchthalten des zu behandelnden Hautareals während der gesamten Einwirkzeit und das mechanische Abwischen des Hautareals mit dem Tupfer, zu keiner Verbesserung des mikrobiellen Wirksamkeit gegenüber dem reinen Aufsprühen der erfindungsgemäßen Zusammensetzung führen.

Da in der Zusammensetzung gemäß Formulierung 7 Isopropanol 100 % verwendet wird und dieser eine geringere antimikrobielle Wirkung hat als Isopropanol 70 % wurde die erfindungsgemäße Zusammensetzung aus der Druckgaspackung auch mit Isopropanol 100 % aus einem Pumpspraysystem verglichen. Erwartungsgemäß zeigte Isopropanol 100 % im Vergleich zum Referenzprodukt (Isopropanol 70 %) eine geringere antimikrobielle Wirkung. In Form der erfindungsgemäßen Zusammensetzung als Druckgasspray appliziert, zeigte der verwendete Isopropanol 100 % diesen Nachteil überraschenderweise nicht mehr.

Es wurde weiters gezeigt, dass auch die Kältewirkung des reinen Kältemittels eine gewisse geringe keimreduzierende Wirkung hat. Diese erhöht bei der erfindungsgemäßen Zusammensetzung die Wirkung des Isopropanols in synergistischer Weise. Daraus resultiert für die erfindungsgemäße Zusammensetzung eine mikrobielle Wirksamkeit, die der aller drei Vergleichsprodukte überlegen ist.

Insgesamt zeigen die Versuche damit, dass die erfindungsgemäße, synergistische Kombination von Antiseptikum, Kältemittel, Wirkverstärker und Druckgassystem zu einer antimikrobiellen Wirksamkeit führt, bei der die Nachteile der Verwendung von reinem Isopropanol 100 % und des Verzichts auf den Auftrag mittels Tupfer nicht nur kompensiert werden, sondern bei der eine Überlegenheit in der Wirksamkeit gegenüber allen Vergleichspräparaten erzielt wird.

### Nachweise der zusätzlichen mikrobiellen Wirksamkeit der Wirkverstärker:

Die für die Anwendung der Zubereitung relevante, talgdrüsenarme menschliche Haut ist mikrobielle relativ schwach besiedelt (z.B. an den Armen 10² - 10³ KbE/cm²). Aus der geringen Ausgangskeimzahl resultieren zwingend auch relativ geringe erreichbare Reduktionsfaktoren (siehe Werte aus Tabelle 2). Mikrobiologische Tests auf der Haut sind daher in erster Linie relevant für die Beurteilung eines verwendungsfertigen Produktes. Um im Rahmen der Produktentwicklung verschiedene Formulierungsvarianten hinsichtlich Ihrer Wirksamkeit zu unterscheiden, werden üblicherweise in-vitro Tests verwendet, insbesondere der oben genannte quantitative Suspensionstest mit oder ohne biotopangepasster Belastung, gemäß der Standardmethode der Deutschen Gesellschaft für Hygiene und Mikrobiologie (DGHM). Dieser Test, der zur Erhöhung der diskriminierenden Eigenschaften mit hoher Belastung durchgeführt wird, wurde deshalb verwendet, um die mikrobielle Wirksamkeit der erfindungsgemäßen Zubereitungen mit und ohne Wirkverstärkern zu vergleichen.

Als Prüfpräparate werden in diesem Test nur die antiseptischen Wirkstoffe der erfindungsgemäßen Zubereitungen ohne Kältemittel und nicht in Form eines Druckgassprays eingesetzt.

Folgende antiseptische Mischungen wurden getestet:

| | |
|---|---|
| I30 110001 | Isopropanol |
| I30 110002 | Isopropanol + 1 % 1,3 Butandiol |
| I30 110003 | Isopropanol + 5 % 1,3 Butandiol |
| I30 110004 | Isopropanol + 1 % Äpfelsäure |
| I30 110005 | Isopropanol + 5 % Äpfelsäure |

Da beim quantitativen Suspensionstest hohe Konzentrationen von wirksamen Antiseptika zu einer vollständigen Abtötung der verwendeten Mikroorganismen führen, wird von den Zubereitungen eine Verdünnungsreihe hergestellt, um zwischen unterschiedlich starken Wirksamkeiten diskriminieren zu können. Die Daten in der folgenden Tabelle 3 beziehen sich auf den Verdünnungsschritt 1:4 (Produktkonzentration: 25 %) und eine Einwirkzeit von 15 sec. Als Testkeim wurde gemäß der DGHM Vorgabe das Bakterium Pseudomonas aeruginosa verwendet.

Ziel des Versuchs war es, zu untersuchen, ob Zusätze von 1,3-Butandiol und Äpfelsäure als Wirkverstärker die antimikrobielle Wirksamkeit von Isopropanol verstärken.

Die Ergebnisse werden in Form logarithmischer Reduktionsfaktoren (RF-Wert) angegeben. Sie stellen die Abnahme der Keimzahl in der Suspension bezogen auf die Einsatzkonzentration dar. Die Einwirkzeit betrug bei allen Versuchen 15 sec. Folgende RF-Werte wurden gefunden:

**Tabelle 3 - Wirkverstärker**

| Testprodukt | RF-Wert gegenüber P. aeruginosa |
|---|---|
| Konzentration des Testprodukts: | 25% |
| I30 110001 | |
| Isopropanol | 4,43 |
| I30 110002 | |
| Isopropanol + 1 % 1,3-Butandiol | 5,38 |
| I30 110003 | |
| Isopropanol + 5 % 1,3-Butandiol | 3,74 |
| I30 110004 | |
| Isopropanol + 1 % Äpfelsäure | >7,22 |
| I30 110005 | |
| Isopropanol + 5 % Äpfelsäure | >7,22 |

Entsprechend der Forderung der DGHM besteht ein Testprodukt den Test, wenn der erzielte RF-Wert für Bakterien >= 5 ist.

### Die Fragestellung des Versuchs wird durch die Ergebnisse wie folgt beantwortet:

Alle 5 Testprodukte entsprechen in unverdünnter Form und bei einem Verdünnungsfaktor von 1 : 2 den Testanforderungen der DGHM. Erst bei einem Verdünnungsfaktor von 1 : 4 diskriminiert die Methode zwischen den Testformulierungen. Bei 1,3-Butandiol ist eine gewisse Wirkverstärkung ersichtlich. Äpfelsäure zeigt bereits in geringer Konzentration eine signifikante und vermutlich klinisch relevante Wirkverstärkung. Alle beobachteten Wirkungen treten bereits nach einer sehr kurzen Einwirkzeit von 15 sec auf.

Eine Überlegenheit im quantitativen Suspensionstest wird nach breit akzeptierter, wissenschaftlicher Lesart so interpretiert, dass die überlegene Formulierung auch in der endgültigen Darreichungsform und in der klinischen Praxis eine überlegene Wirkung aufweist, selbst wenn diese sich mit den standardisierten Test auf der Haut nicht nachweisen lässt. Die praxisnahe Prüfung an Probanden sieht z.B. keine Verdünnung der Testformulierungen vor und wertet die Versuche nach definierten Einwirkzeiten aus. Eine Überlegenheit wird in diesem Fall in folgenden Dimensionen angenommen: Schnelligkeit der Wirkung, Wirksamkeit gegen weniger empfindlichen Problemkeimen, Wirksamkeit unter ungünstigen Umgebungsbedingungen, z.B. blutende, verschmutzte, eitrige Wundareale.

### Nachweis der kühlenden-schmerzreduzierenden Wirkung:

Auch die Wirkung der erfindungsgemäßen Zusammensetzung zur Reduktion der mit Nadelstichen oder Hautpunktionen verbundenen Schmerzempfindung wurde mit Formulierung 7 und mit Formulierung 2 geprüft, wobei allerdings aus ethischen Gründen auf eine Durchtrennung der Haut verzichtet wurde. Der Schmerzreiz wurde mit einer stumpfen Kanüle mit einem Außendurchmesser 0,45 mm durch Drücken auf die Haut in einem Winkel von 90° erzeugt. Durch Einbau der Kanüle in einen handelsüblichen Kugelschreiber wurde ein provisorisches Tactometer konstruiert. Der Kugelschreiber wurde mittels eines Stativs senkrecht auf die Unterarmhaut aufgesetzt und die Kanüle auf die Haut gedrückt, bis der Proband eine unangenehme Druckempfindung signalisierte. Gemessen wurde der Hub des Kugelschreibertasters bis zum Eintritt der unangenehmen Druckempfindung in mm. Der Versuch wurde an 10 Probanden jeweils am rechten und linken Unterarm mit und ohne vorherige Applikation des erfindungsgemäßen Sprays durchgeführt. Die Applikation erfolgte in der gleichen Weise, wie bei der Messung der Hauttemperatur beschrieben. Das Versuchsergebnis ist der nachfolgenden Tabelle 4 zu entnehmen:

**Tabelle 4 - Kühlende-schmerzreduzierende Wirkung**

| | ohne Vorbehandlung | nach Vorbehandlung mit Formulierung 2 und 7 für 2 sec aus 10 cm Entfernung |
|---|---|---|
| Hub des Kugelschreibertasters in mm (Ø aus n=10) | 3,5 mm | 5,0 mm |

Der gemessene größere Hub des Kugelschreibertasters bei einer Vorbehandlung mit dem erfindungsgemäßen Spray bedeutet, dass die Kanüle tiefer in die Haut gedrückt werden konnte, bis ein Schmerzreiz auftrat, dass also die Schmerzempfindlichkeit verringert war.

### Vergleich der Kühlwirkung von verschiedenen Kältesprays:

Zusammensetzungen der untersuchten Kältesprays:
TZ_13/1: Beispiel 1 der DE 1467854: Ethanol : Freon 50 : 50
TZ_13/2: Beispiel 3 der DE 1467854: Ethanol : Freon 20 : 80
TZ_13/3: Beispiel "Formulierung 2": Isopropanol : Alkane 30 : 70

| Muster | Ethanol % m/m | Freon % m/m | Isopropanol % m/m | n-Pentan % m/m | n-Butan % m/m |
|---|---|---|---|---|---|
| TZ_13/1 | 50 | 50 | | | |
| TZ_13/2 | 20 | 80 | | | |
| TZ_13/3 | | | 30 | 20 | 50 |

Die Kühlwirkung dieser drei Kältesprays wurde unter folgenden standardisierten Bedingungen gemessen: Der Inhalt der Kältesprays wurde aus einer Entfernung von 10 cm auf eine 3,4 mm dicke, senkrecht stehende, weiße Polypropylen Platte aufgesprüht. Jeweils nach einem Sprühstoß von 2 Sekunden wurde der Temperaturverlauf an der Plattenoberfläche über 30 Sekunden mit einem IR-Thermometer (Ebro TFI 550) aus 25 cm Entfernung in Zeitintervallen von 5 Sekunden gemessen. Die exakte Menge an Kältemittel wurde durch Rückwiegen der Sprühdose bestimmt, wobei bei den Gasgemischen Butan/Pentan/Isopropanol etwa 1,5 g und bei den Gemischen Freon/Ethanol, bedingt durch den höheren Doseninnendruck, etwa 2,5 g pro Sprühstoß von 2 Sekunden entnommen wurde. Um die Abstände bei jeder Messung reproduzierbar zu gestalten, wurden Platte, Dose und IR-Thermometer mit Stativklemmen fixiert. Die Platte wurde vor jeder Messung auf 20° C temperiert die angegebenen Messwerte stellen Mittelwerte aus drei Einzelmessungen dar.

Die nachfolgende Tabelle bzw. das Diagramm 1 zeigen die unter oben beschriebenen Bedingungen gemessene Kältewirkung der drei Kältesprays:

| Zeit nach Ende des Sprühvorgangs (sec) | TZ_13/1 [°C] | TZ_13/2 [°C] | TZ_13/3 [°C] |
|---|---|---|---|
| 0 | 7,6 | 8,3 | - 14,9 |
| 5 | 15,4 | 11,4 | 3,5 |
| 10 | 16,6 | 13,5 | 8,6 |
| 15 | 17,1 | 15,1 | 11,2 |
| 20 | 17,2 | 15.8 | 12,3 |
| 25 | 17,5 | 16.2 | 13,2 |
| 30 | | 16,4 | |

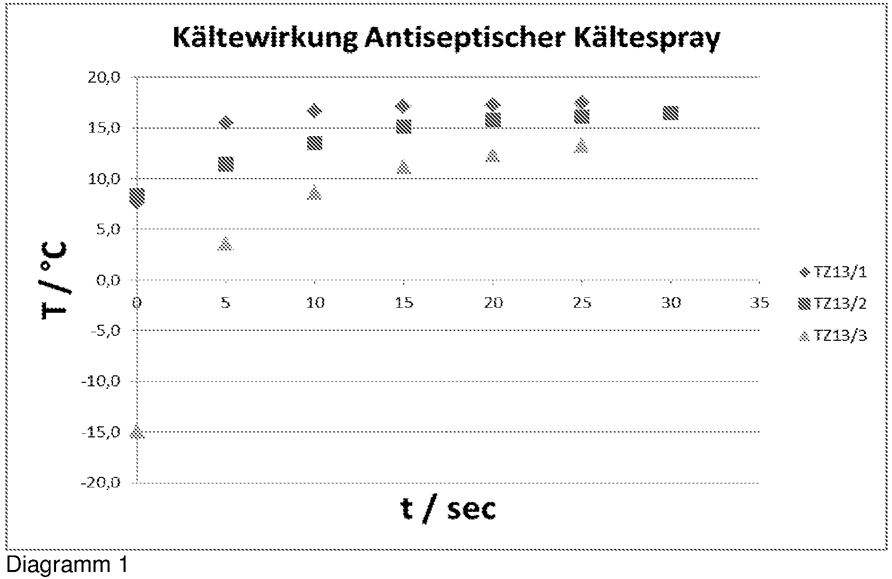

Die Tabelle und Diagramm 1 zeigen, dass die Muster TZ_13/1 und TZ_13/2 gemäß der DE 1467854 eine minimale Temperatur von ca. 8 °C erreichen und dass diese Temperatur bereits nach ca. einer Sekunde wieder auf einen unwirksamen Wert von > 10 °C ansteigt. Demgegenüber lässt sich mit dem erfindungsgemässen Spray entsprechend TZ_13/3 (Formulierung 2) der vorliegenden Anmeldung eine Temperatur von - 15 °C erreichen und die Temperatur bleibt für etwa 13 Sekunden unterhalb des kritischen Wertes von 10 °C.

Der oben gezeigte Effekt trat auf, obwohl die Auftragsmenge bei den Mustern TZ_13/1 und TZ_13/2 um etwa 60 % höher war, als beim Muster TZ_13/3. In weiteren Versuchen wurde gezeigt, dass die erreichbare Kältewirkung weitgehend unabhängig von der Menge an Kältespray ist, die aufgetragen wird. Die minimal erreichbare Temperatur bei einer von 2 Sekunden auf 10 Sekunden verlängerten Sprühzeit beträgt bei Muster TZ_13/1 unverändert 8 °C, bei Muster TZ_13/2 hat se den etwas reduzierten Wert von ca. 5 °C. Mit dem erfindungsgemässen Spray TZ_13/3 gemäß der Formulierung 2 wurde unter gleichen Bedingungen ein Wert von -20 °C errecht.

Ein weiterer Nachteil von Freonen, wie sie z.B. in der DE 1467854 verwendet werden, ist, wie erwähnt, der hohe Druckaufbau in der Druckgaspackung, was dazu führt, dass beim Austritt aus der Ventildüse ein sehr feines Aerosol mit einer geringen mittleren Tröpfchengröße entsteht. Die nachfolgende Tabelle zeigt die Doseninnendrücke für die beiden oben genannten Ausführungsbeispiele TZ_13/1 und TZ_13/2 der DE 1467854 im Vergleich zum Beispiel Formulierung 2 der vorliegenden Anmeldung:

| Muster | Druck bar (20 °C) |
|---|---|
| TZ_13/1 (DE 1467854): | 4,26 |
| TZ_13/2 (DE 1467854): | 5,18 |
| TZ_13/3 (Formulierung 2): | 1,30 |

## Patentansprüche

1. Antiseptische Zusammensetzung zum Aufbringen auf die menschliche Haut in Form einer topisch applizierbaren Sprayformulierung, enthaltend
- einen einzigen antiseptischen Wirkstoff oder eine antiseptische Mischung aus zumindest zwei antiseptischen Wirkstoffen, ausgewählt aus kurzkettigen, 3 C-Atomen enthaltenden, einwertigen Alkoholen aus der Gruppe von Propan-1-ol oder Propan-2-ol (Isopropanol), sowie
- ein einziges Kältemittel oder eine Kältemittel-Mischung aus zumindest zwei Kältemitteln, zur vorübergehenden Reduktion der Schmerzempfindlichkeit der Haut, wobei das Kältemittel oder die Kältemittel-Mischung physikalisch durch Verdampfung kühlt und eine Absenkung der Hautoberflächen-Temperatur bewirkt,
wobei die antiseptischen Wirkstoffe und die Kältemittel voneinander verschieden bzw. unterschiedliche Substanzen sind,
und enthaltend einen, die antiseptische Wirkung verstärkenden, Wirkverstärker, der von den antiseptischen Wirkstoffen und den Kältemitteln unterschiedlich ist, wobei der Wirkverstärker Wasser ist,
wobei der/die antiseptischen Wirkstoffe, das/die Kältemittel und der Wirkverstärker untereinander vollständig mischbar sind,
und wobei die Zusammensetzung frei ist von weiteren therapeutisch wirksamen Bestandteilen, insbesondere frei von Antibiotika, Antimykotika und/oder Virostatika oder Viruziden, und frei ist von weiteren Exzipienten, Hilfsstoffen und/oder Zusatzstoffen, insbesondere frei von viskositätserhöhenden und/oder filmbildenden Polymeren, Stärken, Zuckern, Zuckeralkoholen, Fetten, Ölen, Wachsen, Aromastoffen und/oder längerkettigen, insbesondere geradkettigen, Fettsäuren mit mehr als 10 C-Atomen,
und wobei
- der/die antiseptische(n) Wirkstoff(e) in einer Menge von insgesamt 5 bis 40 Gew.-%, vorzugsweise 10 bis 30 Gew.-%, bezogen auf die gesamte Zusammensetzung,
- das/die Kältemittel in einer Menge von insgesamt 60 bis 95 % Gew.-%, vorzugsweise 70 bis 90 Gew.-%, bezogen auf die gesamte Zusammensetzung,
- und der Wirkverstärker Wasser in einer Menge von insgesamt 0,1 bis 10 Gew.-%, bezogen auf die Menge der antiseptischen Wirkstoffe, enthalten ist/sind.

2. Antiseptische Zusammensetzung nach Anspruch 1, **dadurch gekennzeichnet, dass** die Zusammensetzung bei Raumtemperatur (25 °C) in zumindest teilweise flüssiger Form, in einem Behältnis bei einem gegenüber dem Umgebungsdruck (Normaldruck) erhöhten Druck vorliegt, insbesondere in druckverflüssigter Form in einer Druckgaspackung bzw. als Druckgassystem oder in einem BOV (Bag-on-Valve)-System, wobei der Druck in der Druckgaspackung zwischen 1 und 2 bar (0 - 1 bar Überdruck), besonders bevorzugt zwischen 1,25 und 1,75 bar (0,25 - 0,75 bar Überdruck) liegt.

3. Antiseptische Zusammensetzung nach Anspruch 1 oder 2, **dadurch gekennzeichnet, dass** der antiseptische Wirkstoff bzw. die antiseptische Mischung eine rasche antiseptische Wirksamkeit aufweist(en), bei der die Keimreduktion auf der Haut nach 60, vorzugsweise 30, Sekunden Einwirkzeit nicht signifikant schlechter ist als eine in einem mit ansonsten gleichen Parametern durchgeführten Referenzverfahren mit 70 Vol.-% Isopropanol nach 60 Sekunden erreichte Keimreduktion, bzw. bei der die in einem mikrobiologischen Test auf der Haut, gemäß DGHM Standardmethode 13, Hautdesinfektion - praxisnaher Versuch mit Probanden, gemessenen logarithmischen Reduktionsfaktoren nach 60, vorzugsweise 30, Sekunden Einwirkzeit nicht statistisch signifikant kleiner sind als die in einem mit ansonsten gleichen Parametern durchgeführten Referenzverfahren für das Referenzprodukt Isopropanol 70 % V/V nach 60 Sekunden gemessenen Reduktionsfaktoren.

4. Antiseptische Zusammensetzung nach einem der vorangehenden Ansprüche, **dadurch gekennzeichnet, dass** die antiseptische Zusammensetzung eine rasche antiseptische Wirksamkeit aufweist, bei der die Keimreduktion auf der Haut bereits nach 60, vorzugsweise 30, Sekunden Einwirkzeit nicht signifikant schlechter ist als eine in einem mit ansonsten gleichen Parametern durchgeführten Referenzverfahren mit 70 Vol.-% Isopropanol nach 60 Sekunden erreichte Keimreduktion, bzw. bei der die in einem mikrobiologischen Test auf der Haut, gemäß DGHM Standardmethode 13, Hautdesinfektion - praxisnaher Versuch mit Probanden, gemessenen logarithmischen Reduktionsfaktoren bereits nach 60, vorzugsweise 30, Sekunden Einwirkzeit nicht statistisch signifikant kleiner sind als die in einem mit ansonsten gleichen Parametern durchgeführten Referenzverfahren für das Referenzprodukt Isopropanol 70 % V/V nach 60 Sekunden gemessenen Reduktionsfaktoren.

5. Antiseptische Zusammensetzung nach einem der vorangehenden Ansprüche, **dadurch gekennzeichnet, dass** das einzige Kältemittel oder die Kältemittel-Mischung eine Siedetemperatur zwischen +20 °C und -20 °C aufweist und/oder dass die einzelnen Komponenten einer Kältemittel-Mischung jeweils eine Siedetemperatur zwischen +37 °C und -50 °C aufweisen.

6. Antiseptische Zusammensetzung nach einem der vorangehenden Ansprüche, **dadurch gekennzeichnet, dass** das einzige Kältemittel oder die Kältemittel-Mischung eine bei 21.85°C (295 K) gemessene Verdampfungsenthalpie von mindestens 250 kJ/kg besitzt.

7. Antiseptische Zusammensetzung nach einem der vorangehenden Ansprüche, **dadurch gekennzeichnet, dass** nach einer 2 Sekunden andauernden Sprayapplikation der Zusammensetzung, bei der auf einen Hautbereich am Unterarm mit einer Größe von 10 cm² eine Menge von 150 - 200 mg der Zusammensetzung / cm² aufgetragen wird, eine Abkühlung der Hautoberfläche auf eine Temperatur zwischen 10° und -5°C, insbesondere zwischen 5° und 0° C, bewirkbar ist.

8. Antiseptische Zusammensetzung nach einem der vorangehenden Ansprüche, **dadurch gekennzeichnet, dass** das/die Kältemittel ein verflüssigtes Treibgas, insbesondere ein Flüssiggas aus leicht verflüssigbaren Kohlenwasserstoff-Verbindungen, ist
und/oder das/die Kältemittel ausgewählt ist/sind aus der Gruppe von n-Propan, n-Butan, iso-Butan, n-Pentan, n-Hexan, Chlorethan oder Distickstoffmonoxid,
und/oder dass das einzige Kältemittel oder die Kältemittel-Mischung gleichzeitig als Treibmittel fungiert,
und/oder dass die Kältemittel bzw. die Zusammensetzung frei von Freonen bzw. halogenierten, insbesondere fluorierten, Kohlenwasserstoffen ist,
und/oder dass das/die Kältemittel eine Mischung von n-Pentan und n-Butan ist, insbesondere in einem Gew%-Verhältnis von 15-25 : 45-55, vorzugsweise 20 : 50.

9. Antiseptische Zusammensetzung nach einem der vorangehenden Ansprüche, **dadurch gekennzeichnet, dass** der Gehalt an Rückständen nach einer Verdampfung nach der Sprayapplikation maximal 4 Gew.-%, vorzugsweise maximal 2 Gew.-%, bezogen auf die gesamte Zusammensetzung, beträgt,
und/oder
dass die Zusammensetzung nach der Sprayapplikation auf der menschlichen Haut rückstandsfrei verdampft bzw. dass in der Zusammensetzung ausschließlich rückstandsfrei verdampfende Stoffe enthalten sind.

10. Antiseptische Zusammensetzung nach einem der vorangehenden Ansprüche, **dadurch gekennzeichnet, dass** die Zusammensetzung während und nach der Applikation flüssig und nicht schäumend ist.

11. Druckgaspackung bzw. Druckgassystem, beispielsweise Sprühdose, enthaltend die antiseptische Zusammensetzung nach einem der vorangehenden Ansprüche, in bei Raumtemperatur (25 °C) zumindest teilweise flüssiger Form, insbesondere in druckverflüssigter Form, bei einem gegenüber dem Umgebungsdruck (Normaldruck) um 0 - 1 bar erhöhten Druck.

12. Antiseptische Zusammensetzung nach einem der vorangehenden Ansprüche, zur Verwendung bei der Vorbeugung und Vermeidung von Nadelstichschmerzen bzw. der prophylaktischen Schmerzreduktion vor Hautpunktionen, durch topische Sprayapplikation auf die Haut maximal 60, vorzugsweise maximal 30, Sekunden vor einer Punktion.

13. Antiseptische Zusammensetzung nach Anspruch 12, wobei die Zusammensetzung durch eine 1 bis 5 Sekunden, vorzugsweise 2 Sekunden, andauernde Sprayapplikation auf einen lokalen Hautbereich, insbesondere mit einer Größe von 5 bis 20 cm², vorzugsweise ca. 10 cm², aufgebracht und dabei insgesamt eine Menge von 150 bis 250 mg der Zusammensetzung / cm² aufgetragen wird.

## Claims

1. Antiseptic composition for application to human skin in the form of a topically applicable spray formulation, containing
- a sole antiseptic active ingredient or an antiseptic compound made of at least two active ingredients, chosen from short-chain simple alcohols containing 3 carbon atoms from the group of propan-1-ol or propan-2-ol (isopropyl), and
- a sole cooling agent or a cooling agent compound made of at least two cooling agents, for temporarily reducing the pain sensitivity of the skin, wherein the cooling agent or cooling agent compound physically cools by means of vaporisation and effects a reduction in the skin surface's temperature,
wherein the antiseptic active ingredients and the cooling agent are mutually separate or different substances,
and containing an adjuvant intensifying the antiseptic effect, which is different to the antiseptic active ingredients and the cooling agent, wherein the adjuvant is water,
wherein the antiseptic active ingredient(s), the cooling agent(s) and the adjuvant can be completely mixed with one another,
and wherein the composition is free from other therapeutically effective components, particularly free from antibiotics, antifungals and/or virostatic agents or virucides, and is free from other excipients, auxiliary substances and/or additives, particularly free from viscosity-enhancing and/or film-forming polymers, starches, sugars, sugar alcohols, fats, oils, waxes, flavourings and/or longer-chain, particularly straight-chain, fatty acids with more than 10 carbon atoms,
and wherein
- the antiseptic active ingredient(s) is/are contained in a quantity totalling 5 to 40 wt.-%, preferably 10 to 30 wt.-%, in relation to the overall composition,
- the cooling agent(s) is/are contained in a quantity totalling 60 to 95 wt.-%, preferably 70 to 90 wt.-%, in relation to the overall composition,
- and the adjuvant water is contained in a quantity totalling 0.1 to 10 wt.-%, in relation to the quantity of antiseptic active ingredients.

2. The antiseptic composition according to claim 1, **characterised in that** the composition exists at room temperature (25 °C) in at least a partially liquid form, in a container with a pressure being increased in comparison to the ambient pressure (normal pressure), particularly in pressure liquefied form in an aerosol dispenser or as a pressure gas system or in a BOV (bag-on-valve) system, wherein the pressure in the aerosol dispenser is between 1 and 2 bar (0 - 1 bar upper pressure), particularly preferably between 1.25 and 1.75 bar (0.25 - 0.75 bar excess pressure).

3. The antiseptic composition according to claim 1 or 2, **characterised in that** the antiseptic active ingredient or antiseptic compound has a rapid antiseptic effectivity, with which the germ reduction on the skin after 60, preferably 30, seconds of exposure time is not significantly poorer than a germ reduction being achieved after 60 seconds in a reference procedure with 70 vol% carried out with otherwise identical parameters, or with which the logarithmic reduction factors being measured after 60, preferably 30, seconds of exposure time in a microbiological test on the skin, according to DGHM Standard Method 13, Skin antiseptics - simulated-use test with volunteers, are not statistically significantly smaller than the reduction factors measured after 60 seconds in a reference procedure for the reference product isopropyl 70 v/v % being carried out with otherwise identical parameters.

4. The antiseptic composition according to one of the preceding claims, **characterised in that** the antiseptic composition has a rapid antiseptic effectivity, with which the germ reduction on the skin after just 60, preferably 30, seconds of exposure time is not significantly poorer than a germ reduction being achieved after 60 seconds in a reference procedure with 70 vol% carried out with otherwise identical parameters, or with which the logarithmic reduction factors being measured after 60, preferably 30, seconds of exposure time in a microbiological test on the skin, according to DGHM Standard Method 13, Skin antiseptics - simulated-use test with volunteers, are not statistically significantly smaller than the reduction factors measured after 60 seconds in a reference procedure for the reference product isopropyl 70 v/v % being carried out with otherwise identical parameters.

5. The antiseptic composition according to one of the preceding claims, **characterised in that** the sole cooling agent or the cooling agent compound has a boiling temperature between +20 °C and -20 °C and/or that the individual components of a cooling agent compound each have a boiling temperature between +37 °C and -50 °C.

6. The antiseptic composition according to one of the preceding claims, **characterised in that** the sole cooling agent or the cooling agent compound has an evaporation enthalpy of at least 250 KJ/kg measured at 21.85°C (295 K).

7. The antiseptic composition according to one of the preceding claims, **characterised in that** after a spray application of the composition lasting 2 seconds, during which a quantity of 150 - 200 mg of the composition / cm² is applied to a skin area on the forearm with a size of 10 cm², a cooling of the skin surface to a temperature between 10 ° and -5 °C, particularly between 5 ° and 0 °, can be effected.

8. The antiseptic composition according to one of the preceding claims, **characterised in that** the cooling agent(s) is a liquefied propellant, particularly a liquid gas made of easily liquefiable hydrocarbon compounds
and/or the cooling agent (s) is/are chosen from the group of n-propane, n-butane, isobutane, n-pentane, n-hexane, chloroethane or nitrous oxide,
and/or that the single cooling agent or the cooling agent compound at the same time acts as a propellant,
and/or that the cooling agent or the composition is free from freones or halogenated, particularly fluorinated, hydrocarbons,
and/or that the cooling agent(s) is a mixture of n-pentane and n-butane, particularly in a wt-% ration of 15-25 : 45-55, preferably 20 : 50.

9. The antiseptic composition according to one of the preceding claims, **characterised in that** the content of residue after a vaporisation after the spray application is a maximum 4 wt.-%, preferably a maximum 2 wt.-%, in relation to the entire composition,
and/or
that the composition after the spray application on human skin vaporises without residue or that only residue-free evaporating substances are contained in the composition.

10. The antiseptic composition according to one of the preceding claims, **characterised in that** the composition is liquid and non-foaming during and after application.

11. An aerosol dispenser or pressure gas system, for example a spray can, containing the antiseptic composition according to one of the preceding claims, in an at least partially liquid form at room temperature (25 °C), particularly in a pressure liquefied form, at a pressure of 0 - 1 bar more than the ambient pressure (normal pressure).

12. The antiseptic composition according to one of the preceding claims, for use to prevent and avoid pinprick pains or for the prophylactic pain reduction before skin punctures, by means of topical spray application on the skin no more than 60, preferably no more than 30, seconds before a puncture.

13. The antiseptic composition according to claim 12, wherein the composition is applied by means of a spray application lasting 1 to 5 seconds, preferably 2 seconds, to a local skin area, particularly with a size of 5 to 20 cm², preferably approx. 10 cm², and thereby a total quantity of 150 to 250 mg of the composition / cm² is applied.

## Revendications

1. Composition antiseptique destinée à être appliquée sur la peau humaine sous la forme d'une formulation topique applicable par pulvérisation contenant
- un agent antiseptique unique ou un mélange antiseptique composé d'au moins deux agents antiseptiques, choisis parmi les alcools monovalents à chaîne courte ayant 3 atomes de C provenant du groupe du propan-1-ol ou du propan-2-ol (isopropanol), et
- un réfrigérant unique ou un mélange réfrigérant composé d'au moins deux réfrigérants, pour réduire temporairement la sensibilité de la peau à la douleur, le réfrigérant ou le mélange réfrigérant se refroidissant physiquement par évaporation et provoquant un abaissement de la température à la surface de la peau,
les agents antiseptiques et les réfrigérants étant différents les uns des autres ou étant des substances différentes,
et contenant un adjuvant renforçant l'action antiseptique qui est différent des agents antiseptiques et des réfrigérants, l'adjuvant étant de l'eau,
le/les agent(s) antiseptique(s), le/les réfrigérant(s) et l'adjuvant pouvant être intégralement mélangés ensemble,
et la composition étant exempte d'autres composants thérapeutiquement actifs, en particulier exempte d'antibiotiques, d'antifongiques et/ou de virostatiques ou de virucides, mais aussi exempte d'autres excipients, auxiliaires et/ou additifs, en particulier exempte de polymères augmentant la viscosité et/ou filmogènes, d'amidons, de sucres, de polyols, de graisses, d'huiles, de cires, d'arômes et/ou d'acides gras à chaîne longue, en particulier à chaîne droite ayant plus de 10 atomes de carbone,
et
- le/les agent(s) antiseptique(s) étant contenu(s) dans une quantité globale de 5 à 40 % en poids, de préférence de 10 à 30 % en poids, rapporté à la composition totale,
- le/les réfrigérant(s) étant contenu(s) dans une quantité globale de 60 à 95 % en poids, de préférence de 70 à 90 % en poids, rapporté à la composition totale,
- et l'adjuvant eau étant contenu dans une quantité globale de 0,1 à 10 % en poids, rapporté à la quantité des agents antiseptiques.

2. Composition antiseptique selon la revendication 1, **caractérisée en ce que** la composition, à température ambiante (25 °C), est disponible sous une forme au moins partiellement liquide, dans un récipient à une pression plus élevée par rapport à la pression ambiante (pression normale), en particulier sous une forme liquéfiée sous pression dans un récipient sous pression ou en tant que système à gaz sous pression ou dans un système BOV (aérosol à poche), la pression dans le récipient sous pression étant comprise entre 1 et 2 bar (surpression 0 - 1 bar), de manière particulièrement préférée entre 1,25 et 1,75 bar (surpression 0,25 - 0,75 bar).

3. Composition antiseptique selon la revendication 1 ou 2, **caractérisée en ce que** l'agent antiseptique ou le mélange antiseptique présente une activité antiseptique rapide, dans laquelle la réduction des germes sur la peau après un temps d'action de 60 secondes, de préférence 30, n'est pas significativement pire qu'une réduction des germes obtenue après 60 secondes dans un procédé de référence réalisé selon des paramètres par ailleurs identiques avec 70 % en volume d'isopropanol, ou dans laquelle les facteurs de réduction logarithmiques mesurés lors d'un test microbiologique sur la peau, selon la méthode standard 13 de la DGHM, Désinfection cutanée - expérience pratique sur des sujets, après un temps d'action de 60 secondes, de préférence 30, ne sont pas statistiquement inférieurs aux facteurs de réduction mesurés après 60 secondes dans un procédé de référence réalisé selon des paramètres par ailleurs identiques pour le produit de référence isopropanol à 70 % v/v.

4. Composition antiseptique selon l'une quelconque des revendications précédentes, **caractérisée en ce que** la composition antiseptique présente une activité antiseptique rapide, dans laquelle la réduction des germes sur la peau déjà après un temps d'action de 60 secondes, de préférence 30, n'est pas significativement pire qu'une réduction des germes obtenue après 60 secondes dans un procédé de référence réalisé selon des paramètres par ailleurs identiques avec 70 % en volume d'isopropanol, ou dans laquelle les facteurs de réduction logarithmiques mesurés lors d'un test microbiologique sur la peau, selon la méthode standard 13 de la DGHM, Désinfection cutanée - expérience pratique sur des sujets, après un temps d'action de 60 secondes, de préférence 30, ne sont pas statistiquement inférieurs aux facteurs de réduction mesurés après 60 secondes dans un procédé de référence réalisé selon des paramètres par ailleurs identiques pour le produit de référence isopropanol à 70 % v/v.

5. Composition antiseptique selon l'une quelconque des revendications précédentes, **caractérisée en ce que** le réfrigérant unique ou le mélange réfrigérants présente une température d'ébullition comprise entre +20 °C et -20 °C et/ou **en ce que** les composants individuels d'un mélange réfrigérant présentent respectivement une température d'ébullition comprise entre +37 °C et -50 °C.

6. Composition antiseptique selon l'une quelconque des revendications précédentes, **caractérisée en ce que** le réfrigérant unique ou le mélange réfrigérant possède une enthalpie d'évaporation d'au moins 250 kJ/kg mesurée à 21,85 °C (295 K).

7. Composition antiseptique selon l'une quelconque des revendications précédentes, **caractérisée en ce que**, après avoir appliqué par pulvérisation la composition durant 2 secondes, application au cours de laquelle une quantité de 150 à 200 mg de composition par cm² a été appliquée sur l'avant-bras sur une zone cutanée mesurant 10 cm², il est possible de refroidir la surface de la peau à une température comprise entre 10 °C et -5 °C, en particulier entre 5 °C et 0 °C.

8. Composition antiseptique selon l'une quelconque des revendications précédentes, **caractérisée en ce que** le/les réfrigérant(s) est/sont un gaz propulseur liquéfié, en particulier un gaz liquide provenant de composés hydrocarbonés facilement liquéfiables,
et/ou le/les réfrigérant(s) est/sont choisi(s) dans le groupe du n-propane, du n-butane, de l'isobutane, du n-pentane , du n-hexane, du chloroéthane ou de l'oxyde nitreux,
et/ou **en ce que** le réfrigérant unique ou le mélange réfrigérant fonctionne en même temps comme propulseur,
et/ou **en ce que** les réfrigérants ou la composition sont exempts de fréons ou d'hydrocarbures halogénés, en particulier fluorés,
et/ou **en ce que** le/les réfrigérant(s) est/sont un mélange de n-pentane et de n-butane, en particulier selon un rapport exprimé en % en poids de 15 à 2 : 45 à 55, de préférence de 20 : 50.

9. Composition antiseptique selon l'une quelconque des revendications précédentes, **caractérisée en ce que** la teneur en résidus consécutive à une évaporation après l'application par pulvérisation s'élève au maximum à 4 % en poids, de préférence au maximum à 2 % en poids, rapporté à la composition totale,
et/ou
**en ce que** la composition s'évapore sans résidu sur la peau humaine après l'application par pulvérisation ou **en ce que** la composition contient exclusivement des substances s'évaporant sans résidu.

10. Composition antiseptique selon l'une quelconque des revendications précédentes, **caractérisée en ce que**, pendant et après l'application, la composition est liquide et non moussante.

11. Récipient sous pression ou système à gaz sous pression, par exemple aérosol, contenant la composition antiseptique selon l'une quelconque des revendications précédentes, sous une forme au moins partiellement liquide à température ambiante (25 °C), en particulier sous une forme liquéfiée sous pression, à une pression augmentée de 0 à 1 bar par rapport à la pression ambiante (pression normale).

12. Composition antiseptique selon l'une quelconque des revendications précédentes, destinée à être utilisée pour prévenir et éviter les douleurs dues aux points de suture ou pour réduire à titre prophylactique les douleurs dues aux ponctions cutanées, par application topique par pulvérisation sur la peau durant au maximum 60 secondes, de préférence au maximum 30, avant une ponction.

13. Composition antiseptique selon la revendication 12, la composition étant appliquée par pulvérisation durant 1 à 5 secondes, de préférence 2 secondes, sur une zone cutanée locale, mesurant en particulier 5 à 20 cm², de préférence environ 10 cm², en déposant une quantité globale de 150 à 250 mg de composition par cm².
